# EUROPEAN PATENT SPECIFICATION

(11) **EP 1 857 125 B1**
(45) Date of publication and mention of the grant of the patent: **18.02.2015**
(21) Application number: 06714869.2
(22) Date of filing: 28.02.2006
(51) Int. Cl.: A61L 27/18, C08L 67/04, A61L 27/56, A61L 27/38, C12N 5/20

(54) **METHOD OF FABRICATING SHEET FOR CARTILAGE TISSUE REGENERATION**
VERFAHREN ZUR HERSTELLUNG EINER PLATTE ZUR REGENERATION VON KNORPELGEWEBE
PROCEDE DE FABRICATION D'UNE FEUILLE POUR LA REGENERATION DE TISSU CARTILAGINEUX

(30) Priority: 01.03.2005 JP 2005056171
(43) Date of publication of application: 21.11.2007
(73) Proprietor: GC Corporation, Tokyo 174-8585 (JP); Two Cells Co., Ltd., Hiroshima 732-0811 (JP)
(72) Inventor: KATO, Yukio, Hiroshima, 7320062 (JP); TSUJI, Koichiro, Minami-ku, Hiroshima-shi Hiroshima, 7320811 (JP); YAMANAKA, Katsuyuki, -ku, Tokyo, 1748585 (JP)
(74) Representative: Müller-Boré & Partner Patentanwälte PartG mbB
(86) International application number: PCT/JP2006/303732
(87) International publication number: WO 2006/093137

(56) References cited:
- EP-A2- 1 461 696
- WO-A1-2004/052418
- JP-A- 2003 265 164
- JP-A- 2004 254 655
- JP-A- 2004 350 557
- LIU, SEKIYA, ASAI, TADA, KATO: "biosynthetic response of cultured articular chondrocytes to mechanical vibration" RESEARCH IN EXPERIMENTAL MEDICINE, vol. 200, 5 September 2000 (2000-09-05), pages 183-193, XP002596190
- CHEN G. ET AL.: 'Chondrogenic differentiation of human mensechymal stem cells cultured in a cobweb-like biodegradable scaffold' BIOCHEMICAL AND BIOPHYSICAL RESEARCH COMMUNICATIONS vol. 322, 2004, pages 50 - 55, XP004526699
- DATABASE MEDLINE [Online] ANGELE P. ET AL.: 'Cyclic, mechanical compression enhances chondrogenesis of mesenchymal progenitor cells in tissue engineering scaffolds', XP003003367 Retrieved from STN Database accession no. (15299266) & BIORHEOLOGY vol. 41, no. 3-4, 2004, pages 335 - 346
- DAR A ET AL: "Optimization of Cardiac Cell Seeding and Distribution in 3D Porous Alginate Scaffolds", BIOTECHNOLOGY AND BIOENGINEERING, WILEY & SONS, HOBOKEN, NJ, US, vol. 80, no. 3, 5 November 2002 (2002-11-05), pages 305-312, XP003014569, ISSN: 0006-3592, DOI: 10.1002/BIT.10372

## Description

The present invention relates to a production method of a sheet for regenerating a cartilage tissue, which is used for repairing cartilage damaged by a disease such as osteoarthritis or the like, or an accident.

It has been required at present to realize a regeneration medical treatment for regenerating biotissues and organs which have functional disorders or are dysfunctioned. The regeneration medical treatment is a new medical technique for regenerating biotissues, which cannot be cured by curing ability inherently given to the biotissues, so as to have same shapes and functions as those of original tissues by using a cell, a biomaterial and a cell growth factor.

It is required that a carrier material for regenerating the biotissues has characters such as porosity, biocompatibility, biological absorbency and the like for culturing cells. As the carrier material, a bioabsorbable synthetic high polymer such as polylactic acid, polyglycolic acid and a copolymer of lactic acid and glycolic acid, and a porous carrier material prepared with a natural high polymer such as collagen or the like have been conventionally used (for example, refer to patent documents 1 and 2).

For a regeneration of cartilage, a positive research work has been carried out like those for the other tissues, and contribution to medical treatment of diseases such as osteoarthritis or the like is highly expected. In order to regenerate the cartilage tissue, a porous carrier material is necessary as a step for proliferating chondrocytes or stem cells differentiating to the chondrocytes, and as a support for the biotissues to be formed.

As for a natural high polymer porous material originated in an organism, for example, a sponge and a sheet of collagen are known, and these have hydrophilicity and are remarkably excellent for an interaction with cells. Further, seeding of the cells is easy. However, the material has problems that mechanical strength is low, and handling is hard in a clinical case since it is soft and easily twisted. Therefore, a carrier material comprising the bioabsorbable synthetic high polymer has been widely used.

On the other hand, it has been also known that the same pressure as that in a body is necessary to differentiate the stem cells to the chondrocytes in vitro. When the stem cells are cultured at a normal pressure, the cells are proliferated. However, the cells are dedifferentiated, so that the sufficient amount of the cells for the regeneration cannot be obtained. Further, the cells may lose characteristics as the chondrocytes after the elapse of a long time. Further, a pellet culture method has been also used, where blocks of aggregated cells are produced with centrifugal force and cultured. However, the pellet culture method has a problem that the number of the cells differentiated at a time is small, so that it is remarkably inefficient.

In order to differentiate the stem cells to the chondrocytes, the differentiation has been conventionally carried out by a method for differentiating and culturing under a predetermined pressure, and by a pellet culture method with forming the cell blocks (for example, refer to patent documents 3 to 8). In these methods, culture liquid is supplied while suitably keeping a temperature and a gas concentration and applying the same pressure as the pressure that the cells receive in the body when walking or exercising, and thereby, the cell tissues are cultured with a three-dimensional carrier. However, the conventional pressurizing culture method is mainly carried out under a hydrostatic pressure, and even though pressure changes are periodically given, a continuous pressure must be applied during the culturing or differentiating of the chondrocytes. Thus, there are problems in the control, the cost and the like.

Further, as for the conventional carrier material comprising the bioabsorbable synthetic high polymer, even though it has biological absorbency and excellent mechanical strength, it has high hydrophobicity, so that it is remarkably hard to seed the chondrocytes or the stem cells differentiating to the chondrocytes. So, an effective seeding rate of the cells cannot be obtained, and a large amount of the cells cannot be accumulated in the supporting carrier. Thus, regenerating efficiency of the chondrocytes is low, so that it cannot be practically used. This problem also happens when differentiating or culturing is carried out under the predetermined pressure, and there are problems that the formed chondrocytes have a thin layer and seeding efficiency is low.
Patent Document 1: Japanese Patent Application Laid Open No. 2003-10308
Patent Document 2: Japanese Patent Application Laid Open No. 2004-105046
Patent Document 3: Japanese Patent Application Laid Open No. 2001-238663
Patent Document 4: Japanese Patent Application Laid Open No. 2002-306157
Patent Document 5: Japanese Patent Application Laid Open No. 2002-315566
Patent Document 6: Japanese Patent Application Laid Open No. 2003-169663
Patent Document 7: Japanese Patent Application Laid Open No. 2003-180331
Patent Document 8: Japanese Patent Application Laid Open No. 2003-289851

Liu and al. (Experiment Medicine, 2001, 200:183-193) discloses a method to grow and proliferate chondrocytes in vitro, said method comprising the steps of loading cells into scaffold and then applying a cyclic, mechanical compression during the culture period. The cyclic compression improves the cell re-differentiation.

An objective of the present invention is to provide a production method of a sheet for regenerating a cartilage tissue, which uses a sheet-shaped porous body comprising a biological absorbency synthetic high polymer such as polylactic acid, polyglycolic acid, and a copolymer of lactic acid and glycolic acid, in which chondrocytes can be re-differentiated or stem cells differentiating to chondrocytes can be differentiated without culturing the cells by pressurizing, like as a conventional pressurizing culture, and the cells can be accumulated in a supporting carrier with high efficiency.

The earnest work was carried out in order to solve the above-mentioned problems and, as a result of this, the followings were found to complete the present invention. When a specific magnitude of acceleration is applied to a porous body, after chondrocytes or stem cells differentiating to chondrocytes are seeded on the sheet-shaped porous body comprising a biological absorbency synthetic high polymer, the chondrocytes or the stem cells differentiating to the chondrocytes can be certainly differentiated to the cartilage without applying any acceleration or pressure thereafter, and in addition, the cartilage can be cultured with high seeding efficiency.

That is, the present invention is a production method of a sheet for regenerating a cartilage tissue, the method comprising the steps of; seeding chondrocytes or stem cells differentiating to the chondrocytes on the sheet-shaped porous body comprising the biological absorbency synthetic high polymer; taking the seeded porous body into a culture liquid; applying acceleration of 981 to 9810 m/s² (100 to 1000G) for a predetermined time; and culturing the porous body without applying acceleration thereafter. As for the sheet-shaped porous body comprising the biological absorbency synthetic high polymer, it is preferable that the porous body comprises at least one or more different kinds of homopolymers or copolymers selected from homopolymers or copolymers of L-lactic acid, DL-lactic acid, glycolic acid, ε- caprolactone, polymalic acid and chitosan, where these homopolymers or copolymers have a molecular weight of 40,000 to 500,000. Further, it is preferable that the porous body has hole diameters of 1 to 200µm, a porosity of 5 to 95%, and a thickness of 50 to 500µm.

The production method of the sheet for regenerating the cartilage tissue according to the present invention has the steps of seeding the chondrocytes or the stem cells differentiating to the chondrocytes on the sheet-shaped porous body comprising the biological absorbency synthetic high polymer and applying the predetermined acceleration to the porous body, so that the stem cells can be certainly differentiated to the cartilage without applying any acceleration or pressure thereafter, and in addition, the cartilage can be cultured with high seeding efficiency.

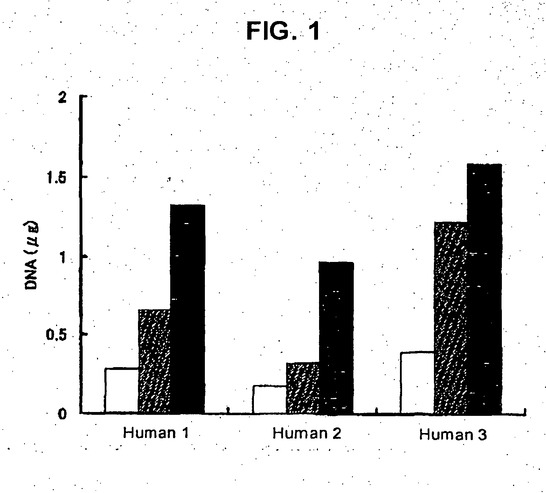

Figure 1 is a bar graph indicating DNA amounts of cartilage differentiated tissues, in the case of a sheet for regenerating a cartilage tissue produced by a method of the present invention being used, the case of a non-centrifugal force application sheet produced without applying the centrifugal force being used, and the case of a cartilage tissue pellet without using a sheet-shaped porous body comprising a biological absorbency synthetic high polymer.

As for a sheet-shaped porous body comprising a biological absorbency synthetic high polymer used in the present invention, it is preferable, from points of strength and safety to an organism, to use a biological absorbency synthetic high polymer selected from a homopolymer or a copolymer of L-lactic acid, DL-lactic acid, glycolic acid, ε- caprolactone, polymalic acid, chitosan.

Further, it is preferable that the molecular amount of the biological absorbency synthetic high polymer structuring the porous body is 40,000 to 500,000. If the molecular amount is less than 40,000, hardness of a filmmay be decreased, and if it is more than 500, 000, the film may become too hard. If a hole diameter of the porous body is less than 1µm, flexibility of the sheet is insufficient, and if it is more than 200µm, a sheet surface is remarkably roughed, so that the seeding rate of the cells may be decreased.

If the porosity of the porous body is less than 5%, there is no effect of the porosity, and flexibility of the sheet is insufficient. If the porosity is more than 95%, the sheet becomes too flexible, so that it is hard to carry out a clinical operation. Further, if the thickness of the sheet of the porous body is less than 200µm, the sheet is thin and easily broken, so that operativity is decreased, and if it is more than 500µm, since the sheet becomes too hard, the operativity may be decreased.

The chondrocytes or the stem cells differentiating to the chondrocytes, which is used in the present invention, can be obtained by directly taking the chondrocytes, or taking the stem cells, which can differentiate to the chondrocytes or have ability to promote repairing of the chondrocytes, where the stem cells are, for example, mesenchymal stem cells, mesenchymal cells, periodontal ligament cells, synovial cells or the like. As for a taking method, a method generally carried out in a medical department can be used without limiting especially. For example, the cells can be taken from a bone marrow and/or a periosteum of a pelvis (an ilium) or a long bone of hand and foot (a femur, a tibia), a bone marrow of an alveolar bone or the like, a periosteum of a palate, a alveolar bone or the like, or the like. In these bones, since the cells can be taken by a simple operation requiring a minimum exfoliation and incision of the skin and muscle, taking from the bone marrow of the alveolar bone or the like, or the periosteum of the palate, the alveolar bone or the like is preferable.

The taken chondrocytes or stem cells differentiating to the chondrocytes are amplifying-cultured in a culture plate for culturing a tissue for 1 to 2 weeks according to a conventional method. As a culture medium for culturing the cells, a suitable culture medium can be used. However, for example, a DMEM culture medium for culturing cells, which contains self serum or fetal bovine serum (FBS), can be preferably used. At this time, when the specific growth factor (for example, bFGF) is added, the mesenchymal stem cells are proliferated with keeeping multiple differentiation potency so as to promote regenerating the cartilage, so that the mesenchymal stem cells have remarkable regenerating ability.

Such the mesenchymal stem cells, which are ultra-amplifying-cultured with keeping the multiple differentiation potency under the existence of the specific growth factor, are exfoliated from the culture plate by a trypsin treatment, and seeded on the sheet-shaped porous body comprising the biological absorbency synthetic high polymer.

Then, the sheet-shaped porous body, which is seeded with the chondrocytes or the stem cells differentiating to the chondrocytes, is taken into the culture liquid, and applied with the acceleration of 981 to 9810 m/s² (100 to 1000G), preferably 1962 to 5886 m/s² (200 to 600G) for a predetermined time by a centrifugal separator or the like. By applying the acceleration, the chondrocytes or the stem cells differentiating to the chondrocytes can be certainly differentiated to the chondrocytes without culturing under the pressurizing condition after that. Further, by the high pressure of the acceleration, the chondrocytes or the stem cells differentiating to the chondrocytes can be invaded into the inside of the sheet-shaped porous body. As a result of this, the layer of the chondrocytes, which is thicker than the conventional layer and has good adhesion with the biological absorbency synthetic high polymer, can be obtained.

At this time, the acceleration may be applied toward the sheet-shaped porous body comprising the biological absorbency synthetic high polymer from the side of the seeded chondrocytes or stem cells differentiating to the chondrocytes, and the acceleration may be applied toward the seeded chondrocytes or stem cells differentiating to the chondrocytes from the side of the sheet-shaped porous body comprising the biological absorbency synthetic high polymer. However, when the acceleration is applied toward the sheet-shaped porous body comprising the biological absorbency synthetic high polymer from the side of the seeded chondrocytes or stem cells differentiating to the chondrocytes, the cells can be easily invaded into the inside of the sheet-shaped porous body. Thus, the high seeding rate of the cells can be obtained, so that it is preferable.

The time of applying the acceleration is changed with the strength of the acceleration, the density of the cells, and conditions of the sheet-shaped porous body comprising the biological absorbency synthetic high polymer to be used. However, the time of applying the acceleration is preferably 30 seconds to 30 minutes. Further, the temperature at the time of applying the acceleration is equal to the general cell culture temperature and not limited especially.

After the acceleration of 981 to 9810 m/s² (100 to 1000G) is applied to the culture liquid having the sheet-shaped porous body comprising the biological absorbency synthetic high polymer which is seeded with the chondrocytes or the stem cells differentiating to the chondrocytes, the sheet-shaped porous body comprising the biological absorbency synthetic high polymer is cultured for 3 to 4 weeks at the normal pressure, by using a culture medium suitable for inducing the differentiation to the cartilage (for example, a culture medium indicated in a document, Science 284, 143-147, 1999). Thereby, the chondrocytes are cultured, or the stem cells are differentiated to the chondrocytes. In the method of the present invention, it is not necessary to apply acceleration and/or pressure at the time of culturing. Example

Hereinafter, the present invention is described concretely with examples, but the present invention is not limited to these examples.

### 1) Taking of a bone marrow liquid from an ilium

Bone marrow liquids were taken from iliums of 3 persons under a negative pressure using a Komiya-type puncture needle, where the 3 persons are under general anesthesia. Then, the taken bone marrow liquids were respectively washed with a 10% FBS and a DMEM culture medium, and the liquids were suspended in the washing liquid and loosened. Then, the liquids were centrifugally separated at 2943 m/s² (300G) for 5 minutes so as to separate the cells, and then, about 7×10⁷ nucleate cells were obtained.

### 2) Culturing of bone marrow-originating mesenchymal stem cells

The 7×10⁷ nucleate cells taken from the marrow liquid were seeded onto a 75cm² culture flask of the DMEM culture medium which was the same as that used in the above-description 1), and cultured under the existence of 5% carbon dioxide at 37°C. After 3 days, the culture medium was changed so as to remove non-adhered cells. After that, the culture medium was changed once 3 days. Then, after 5 days, the bFGF was added to the culture medium at the ratio of 3 ng/ml. As a result of this, the cells were proliferated so as to be approximately confluent after about 10 days. The culture flask was incubated with 0.05% trypsin and 0.2mM MEDTA for 5 minutes so as to isolate the cells. The number of the cells was measured by a Coulter counter (Z1 single, made by Coulter Corporation), and the cells was seeded onto a 75cm² culture flask of the 10% FBS and the DMEM culture medium with the density of 5000 cells/cm². This operation was repeated, and third cells obtained from a second passage culture plate, in which the cells were approximately confluent, were made as mesenchymal cells.

### 3) Producing of a sheet-shaped porous body comprising a biological absorbency synthetic high polymer

A high polymer blend was dissolved with dioxane, and frozen and dried, where the high polymer blend was obtained by mixing poly-L-lactic acid having a molecular weight of 230,000 and a copolymer of DL-lactic acid and glycolic acid having a molecular weight of 250,000 in a predetermined ratio. Then, a sheet-shaped porous body comprising a biological absorbency synthetic high polymer was produced, where the porous body had the average hole diameter of 21µm, the porosity of 65% and the thickness of 250µm.

### 4) Producing of a sheet for regenerating a cartilage tissue

The sheet-shaped porous body comprising the biological absorbency synthetic high polymer was cut to have a diameter of 9mm, and the above-described mesenchymal cells are seeded on the porous body in the confluent state. The acceleration of 3453,12 m/s² (352G) was applied (a radius of 15 cm, 1500 rpm) approximate-vertically toward the sheet-shaped porous body comprising the biological absorbency synthetic high polymer from the side of the seeded mesenchymal cells, where the acceleration was applied for 3 minutes by a centrifugal separator (the product name: a small and desk type centrifugal machine, made by Kokusan Corporation). Then, the cell pellet was cultured at a normal pressure and 37°C for 4 weeks with a cartilage differentiation inducing culture medium (MEM containing 50µg/ml Ascorbic acid 2-phosphate, 100µg/ml Pyruvate, 4.5 g/l D- (+) -glucose, 2mM L-glutamine, 10 ng/ml TGF-β3, 10_⁷M Dexamethason, and 1% ITS+), and then, a sheet for regenerating a cartilage tissue was produced.

In order to grasp the number of the cells in the sample, the total DNA was measured by using PicoGreen dsDNA Quantitation Kit (Molecular Probes, P-7589) made by Molecular Probe Corporation. After washing the sheet for regenerating a cartilage tissue produced by the above-described method with PBS, a Papain solution (300µg/ml Papain, 2mM EDTA, 2mM N-acetylcysteine, 50mM KPB (pH 6.5)) was added, and incubation was carried out at 60°C for 1 hour. The cells were disintegrated by an ultrasonic disintegrator (made by SONIX&MATERIALS Inc., an amplitude scale 30 at Vibra Cell-model 130, 10 sec, on ice). Extracted materials were transferred to tubes, and these were made as samples for measuring DNA. The samples were kept at -30°C until using those. An analytical curve solution was prepared by successively diluting a λ DNA standard solution (100µg/ml) with a Tris-EDTA solution (TE). As for the samples for measuring DNA, solutions which were diluted 5 times and 10 times with 10mM Tris-HCL, 1mM EDTA, pH7.5 (TE) were prepared, and these solutions were arbitrary added at 100µL per well plate to 96 well plates (Nunclon Surface, Nunc 137101). These solutions were lightly stirred, and after 5 minutes, the Ex/Em: 485/535nm (480/520nm) was measured. The DNA amount of each sample was obtained from the standard curve of λ DNA. These results were shown with a black colored painting bar graph in Figure 1.

### 5) Producing of comparison example of a cartilage sheet (a sheet without applying centrifugal force)

The sheet-shaped porous body comprising the biological absorbency synthetic high polymer was cut to have a diameter of 8mm, and the above-described mesenchymal cells are seeded on the porous body in the confluent state. Then, the porous body was cultured without applying a pressure by centrifugal force, and it was cultured at a normal pressure and 37°C for 4 weeks with a cartilage differentiation inducing culture medium (MEM containing 50µg/ml Ascorbic acid 2-phosphate, 100µg/ml Pyruvate, 4.5g/1D-(+)-glucose, 2mM L-glutamine, 10 ng/ml TGF-β3, 10_⁷M Dexamethason, and 1% ITS+), and then, a sheet for regenerating a cartilage tissue was produced.

In order to grasp the number of the cells in the sample, the toatal DNA was measured by using PicoGreen dsDNA Quantitation Kit (Molecular Probes, P-7589) made by Molecular Probe Corporation. After washing the sheet for regenerating a cartilage tissue produced by the above-described method with PBS, a Papain solution (300µg/ml Papain, 2mM EDTA, 2mM N-acetylcysteine, 50mM KPB (pH 6.5)) was added, and incubation was carried out at 60°C for 1 hour. The cells were disintegrated by an ultrasonic disintegrator (made by SONIX&MATERIALS Inc., an amplitude scale 30 at Vibra Cell-model 130, 10 sec, on ice). Extracted materials were transferred to tubes, and these were made as samples for measuring DNA. The samples were kept at -30°C until using those. An analytical curve solution was prepared by successively diluting a λ DNA standard solution (100µg/ml) with a Tris-EDTA solution (TE). As for the samples for measuring DNA, solutions which were diluted 5 times and 10 times with 10mM Tris-HCL, 1mM EDTA, pH7.5 (TE) were prepared, and these solutions were arbitrary added at 100µL per well plate to 96 well plates (Nunclon Surface, Nunc 137101). These solutions were lightly stirred, and after 5 minutes, the Ex/Em: 485/535nm (480/520nm) was measured. The DNA amount of each sample was obtained from the standard curve of λ DNA. These results were shown with a hatched bar graph in Figure 1.

### 6) Producing of a comparison example of a cartilage sheet (a cartilage tissue pellet)

The above-described mesenchymal cells are suspended in the cartilage differentiation inducing culture medium (MEM containing 50µg/ml Ascorbic acid 2-phosphate, 100µg/ml Pyruvate, 4.5 g/l D-(+)-glucose, 2mM L-glutamine, 10 ng/ml TGF-β3, 10_⁷M Dexamethason, and 1% ITS+), and these cells were transferred to a 15 ml centrifugation tube so as to have 300,000 cells per centrifugation tube. The acceleration of 3453,12 m/s² (352G) was applied (a radius of 15 cm, 1500 rpm) approximate-vertically toward the bottom face of the centrifugation tube from the side of the seeded mesenchymal cells, where the acceleration was applied for 3 minutes by a centrifugal separator (the product name: a small and desk type centrifugal machine, made by Kokusan Corporation). Then, the porous body was cultured under a normal pressure and 37°C for 4 weeks with a cartilage differentiation inducing culture medium (MEM containing 50µg/ml Ascorbic acid 2-phosphate, 100µg/ml Pyruvate, 4.5 g/l D- (+)-glucose, 2mM L-glutamine, 10 ng/ml TGF-β3, 10_⁷M Dexamethason, and 1% ITS+). After the cells in the centrifugation tube were seeded, the cell blocks were formed, and after culturing is completed, a cartilage tissue pellet of about 1 mm was produced.

In order to grasp the number of the cells in the sample, the toatal DNA was measured by using PicoGreen dsDNA Quantitation Kit (Molecular Probes, P-7589) made by Molecular Probe Corporation. After washing the cartilage tissue pellet produced by the above-described method with PBS, a Papain solution (300µg/ml Papain, 2mM EDTA, 2mM N-acetylcysteine, 50mM KPB (pH 6.5)) was added, and incubation was carried out at 60°C for 1 hour. The cells were disintegrated by an ultrasonic disintegrator (made by SONIX&MATERIALS Inc., an amplitude scale 30 at Vibra Cell-model 130, 10 sec, on ice). Extracted materials were transferred to tubes, and these were made as samples for measuring DNA. The samples were kept at -30°C until using those. An analytical curve solution was prepared by successively diluting a λ DNA standard solution (100µg/ml) with a Tris-EDTA solution (TE). As for the samples for measuring DNA, solutions which were diluted 5 times and 10 times with 10mM Tris-HCL, 1mM EDTA, pH7.5 (TE) were prepared, and these solutions were arbitrary added at 100µL per well plate to 96 well plates (Nunclon Surface, Nunc 137101). These solutions were lightly stirred, and after 5 minutes, the Ex/Em: 485/535nm (480/520nm) was measured, and the DNA amount of each sample was obtained from the standard curve of λ DNA. These results were shown with an uncolored bar graph in Figure 1.

The followings were found out from Figure 1. In the sheet for regenerating a cartilage tissue, which is obtained by seeding the chondrocytes or the stem cells differentiating to the chondrocytes on the sheet-shaped porous body comprising the biological absorbency synthetic high polymer; taking the seeded porous body into the culture liquid; applying the acceleration of 981 to 9810 m/s² (100 to 1000G) for a predetermined time; and culturing without applying the acceleration to the porous body thereafter, the DNA amount was remarkably high, when compared with that of the non-centrifugal force application sheet, which is not applied with the acceleration, and that of the cartilage tissue pellet without using the sheet-shaped porous body comprising the biological absorbency synthetic high polymer. Thus, regeneration efficiency of the cartilage tissue in the former is excellent.

Further, the sheet for regenerating a cartilage tissue produced by the method of the present invention was transplanted to a subcutaneous part of a back of a nude mouse. After 7 weeks from transplanting the sheet, a sample was extracted, and subjected to a hematoxylin eosin dyeing and a safranin-O dyeing. Further, m-RNA was taken from the sample, and manifestation analyses of a type II collagen and an aggrecan were carried out by the RT-PCR, where the type II collagen and the aggrecan were especially existed in an articular cartilage tissue. As a result of this, it was observed that the sample transplanted to the subcutaneous part of the back of the nude mouse has a surface gloss and a color of opalescence. Further, as a result of the hematoxylin eosin dyeing and the safranin-O dyeing, a small round cell in alveolus and an extracellular matrix having Sufranin-O dyeing property were observed. Further, in the m-RNA sample extracted from the sample, the m-RNA showing the type II collagen and the agrrecan was detected and identified. Thus, it was confirmed that the regenerated tissue was the articular cartilage tissue. Furthermore, as for the obtained sheet for regenerating a cartilage tissue, it was confirmed that the chondrocytes were cultured in the high density state on the surface and the sufficient depth from the surface.

## Claims

1. A production method of a sheet for regenerating a cartilage tissue, the method comprising the steps of;
seeding chondrocytes or stem cells differentiating to the chondrocytes on a sheet-shaped porous body comprising a biological absorbency synthetic high polymer;
taking the seeded porous body into a culture liquid;
applying acceleration of 981 to 9810 m/s² (100 to 1000G) to the porous body for a predetermined time; and
culturing the porous body without applying acceleration thereafter.

2. The production method of the sheet for regenerating a cartilage tissue as claimed in claim 1,
wherein the acceleration is applied toward the sheet-shaped porous body comprising the biological absorbency synthetic high polymer from the side of the seeded chondrocytes or stem cells differentiating to the chondrocytes.

3. The production method of the sheet for regenerating a cartilage tissue as claimed in claim 1,
wherein the acceleration is applied toward the seeded chondrocytes or stem cells differentiating to the chondrocytes from the side of the sheet-shaped porous body comprising the biological absorbency synthetic high polymer.

4. The production method of the sheet for regenerating a cartilage tissue as claimed in any one of claims 1 to 3,
wherein the sheet-shaped porous body comprising the biological absorbency synthetic high polymer comprises at least one or more different kinds of homopolymers or copolymers selected from homopolymers or copolymers of L-lactic acid, DL-lactic acid, glycolic acid, ε-caprolactone, polymalic acid and chitosan, and the homopolymer or the copolymer has a molecular weight of 40,000 to 500,000, and
wherein the porous body has a hole diameter of 1 to 200µm, a porosity of 5 to 95%, and a thickness of 50 to 500µm.

## Patentansprüche

1. Herstellungsverfahren einer Folie für die Regeneration von Knorpelgewebe, wobei das Verfahren die Schritte umfaßt:
Aussäen von Chondrozyten oder Stammzellen, die zu Chondrozyten differenzieren, auf einen folienförmigen porösen Körper, umfassend ein synthetisches Hochpolymer mit biologischem Absorptionsvermögen,
Aufnehmen des besäten porösen Körpers in eine Kulturflüssigkeit,
Anwenden einer Beschleunigung von 981 bis 9810 m/s² (100 bis 1000 G) auf den porösen Körper für eine vorbestimmte Dauer, und
Kultivieren des porösen Körpers ohne danach eine Beschleunigung anzuwenden.

2. Herstellungsverfahren der Folie für die Regeneration von Knorpelgewebe nach Anspruch 1,
wobei die Beschleunigung auf den folienförmigen porösen Körper, umfassend das synthetische Hochpolymer mit biologischem Absorptionsvermögen, von der Seite der besäten Chondrozyten oder Stammzellen, die zu Chondrozyten differenzieren, angewendet wird.

3. Herstellungsverfahren der Folie für die Regeneration von Knorpelgewebe nach Anspruch 1,
wobei die Beschleunigung auf die besäten Chondrozyten oder Stammzellen, die zu Chondrozyten differenzieren, von der Seite des folienförmigen porösen Körpers, umfassend das synthetische Hochpolymer mit biologischem Absorptionsvermögen, angewendet wird.

4. Herstellungsverfahren der Folie für die Regeneration von Knorpelgewebe nach einem der Ansprüche 1 bis 3,
wobei der folienförmige poröse Körper, umfassend das synthetische Hochpolymer mit biologischem Absorptionsvermögen, mindestens eine oder mehrere verschiedene Arten von Homopolymeren oder Copolymeren, ausgewählt aus Homopolymeren oder Copolymeren von L-Milchsäure, DL-Milchsäure, Glycolsäure, ε-Caprolacton, Polyäpfelsäure und Chitosan, umfaßt, und das Homopolymer oder das Copolymer ein Molekulargewicht von 40.000 bis 500.000 aufweist, und
wobei der poröse Körper einen Lochdurchmesser von 1 bis 200 µm, eine Porosität von 5 bis 95% und eine Dicke von 50 bis 500 µm aufweist.

## Revendications

1. Procédé de production d'une feuille pour la régénération d'un tissu cartilagineux, le procédé comprenant les étapes de :
- ensemencer des chondrocytes ou des cellules souches se différenciant en chondrocytes sur un corps poreux en forme de feuille comprenant un polymère élevé synthétique d'absorbance biologique ;
- mettre le corps poreux ensemencé dans un liquide de culture ;
- appliquer une accélération de 981 à 9810 m/s² (100 à 1000 G) sur le corps poreux pendant une durée prédéfinie ; et
- cultiver le corps poreux sans appliquer ensuite d'accélération.

2. Procédé de production d'une feuille pour la régénération d'un tissu cartilagineux selon la revendication 1,
dans lequel l'accélération est appliquée vers le corps poreux en forme de feuille comprenant le polymère élevé synthétique d'absorbance biologique du côté des chondrocytes ensemencés ou des cellules souches se différenciant en chondrocytes.

3. Procédé de production d'une feuille pour la régénération d'un tissu cartilagineux selon la revendication 1,
dans lequel l'accélération est appliquée vers les chondrocytes ensemencés ou les cellules souches se différenciant en chondrocytes du côté du corps poreux en forme de feuille comprenant le polymère élevé synthétique d'absorbance biologique.

4. Procédé de production d'une feuille pour la régénération d'un tissu cartilagineux selon l'une quelconque des revendications 1 à 3,
dans lequel le corps poreux en forme de feuille comprenant le polymère élevé synthétique d'absorbance biologique comprend au moins un ou plusieurs types différents d'homopolymères ou de copolymères choisis parmi les homopolymères ou les copolymères d'acide L-lactique, d'acide DL-lactique, d'acide glycolique, de ε-caprolactone, d'acide polymalitque de de chitosane, et l'homopolymère ou le copolymère a un poids moléculaire de 40 000 à 500 000, et
dans lequel le corps poreux a un diamètre de trou de 1 à 200 µm, une porosité de 5 à 95 %, et une épaisseur de 50 à 500 µm.
